# EUROPÄISCHE PATENTSCHRIFT

(11) **EP 3 626 210 B1**
(45) Veröffentlichungstag und Bekanntmachung des Hinweises auf die Patenterteilung: **23.06.2021**
(21) Anmeldenummer: 19192220.2
(22) Anmeldetag: 19.08.2019
(51) Int. Cl.: A61F 2/52, A61F 2/50

(54) **VERFAHREN ZUR HERSTELLUNG EINER BRUSTPROTHESE**
METHOD FOR MANUFACTURING A BREAST PROSTHESIS
PROCÉDÉ DE FABRICATION D'UNE PROTHÈSE MAMMAIRE

(30) Priorität: 14.09.2018 DE 102018122566
(43) Veröffentlichungstag der Anmeldung: 25.03.2020
(73) Patentinhaber: Amoena Medizin-Orthopädie-Technik GmbH, 83064 Raubling (DE)
(72) Erfinder: Stelter, Nils, 83112 Frasdorf (DE)
(74) Vertreter: Laufhütte, Dieter

(56) Entgegenhaltungen:
- EP-A1- 1 072 239
- JP-A- H05 131 007
- JP-A- H07 328 050
- US-A1- 2012 077 010
- US-A1- 2013 116 786
- US-A1- 2017 367 850

## Beschreibung

Die Erfindung betrifft ein Verfahren zur Herstellung einer Brustprothese.

Brustprothesen werden nach Mastektomien getragen. Anforderungen an die Brustprothesen umfassen insbesondere eine der natürlichen Brust möglichst nahekommenden Form und Haptik sowie ein hoher Tragekomfort und gute Entlüftung mit Wärmeregulierung der anliegenden, oft vernarbten Hautbereiche.

Zur Herstellung derartiger Brustprothesen ist es beispielsweise aus der WO 2013/091720 A1 bekannt, eine Brustprothese dadurch herzustellen, dass eine Gussform mit einer zur Vorderseite einer Brust komplementär geformten Kavität bereitgestellt wird, dass eine vernetzbare Silikonmasse in die Kavität gegossen, in Schichtform gebracht und gehärtet wird, um eine dünne Hautschicht der Brustprothese zu erhalten, dass die mit der Hautschicht belegte Kavität zumindest teilweise mit einer Dispersion von granularem, löslichem Material in einer vernetzbaren Silikonmasse befüllt und die Silikonmasse gehärtet wird, und Auslösen des granularen, löslichen Materials, um einen Prothesenkörper mit einer zellulären Struktur zu erhalten. In der US 2017/367850 A1 wird ein weitergebildetes derartiges Verfahren offenbart, wobei der vernetzbaren Silikonmasse ein suspendiertes Phasenwechselmaterial zugegeben wird, dessen Schmelzpunkt in einem Bereich etwas unterhalb der Körpertemperatur liegt.

Ein Nachteil dieser bekannten Verfahren liegt darin, dass es für das Auslösen des granularen, löslichen Materials aus der gehärteten Silikonmatrix eine dichte Kugelpackung erforderlich ist, damit sich alle Kugeln berühren, um Auswaschkanäle durch das gesamte Volumen zu gewährleisten. Das Porenvolumen des Prothesenkerns lässt sich somit nicht frei wählen. Auch die Porenhomogenität und die Einstellbarkeit der Porengröße ist nicht immer optimal, und das Auswaschen zeitaufwändig. Rückstände des lösbaren Materials lassen sich kaum vermeiden.

Aus der JP H05 131 007 A ist ein Verfahren zur Herstellung einer Brustprothese bekannt, bei dem ein geschwollenes granulares Kunststoffmaterial in eine Kavität einer Gussform eingebracht wird, welches nach Aushärten einen Prothesenkörper bildet, in dem durch anschließendes Erwärmen Poren erzeugt werden. Dabei wird das Kunststoffmaterial durch Erwärmen des Prothesenkörpers auf eine Schrumpfungstemperatur durch Trocknung geschrumpft. Auch aus der US 2013 / 116 786 A1 ist ein Verfahren zur Herstellung einer Brustprothese bekannt, wobei das verwendete poröse thermoplastische Kunststoffgranulat als Füllstoff in seiner ursprünglichen Form im Implantat erhalten bleibt. Bei einem aus der EP 1 072 239 A1 bekannten Herstellungsverfahren für eine Brustprothese wird das verwendete granulare Kunststoffmaterial vor dem Vermischen mit der Silikonmasse verdichtet und dehnt sich durch die Wärmezufuhr beim Aushärten der Silikonmasse aus. Die JP H07 328 050 A offenbart eine Brustprothese, die mit einem Verfahren ähnlich der JP H05 131 007 A hergestellt ist, wobei nur im Bereich einer rückseitigen Abschlusszone und nicht für den kompletten Prothesenkörper poröses Material verwendet wird.

Aufgabe der Erfindung ist es ein gattungsgemäßes Verfahren zur Herstellung einer Brustprothese bereitzustellen, das die oben genannten Nachteile überwindet.

Vor diesem Hintergrund betrifft die Erfindung ein Verfahren zur Herstellung einer Brustprothese, umfassend die folgenden Schritte: (b) Einbringen einer ersten Dispersion von erstem granularem Material in einer vernetzbaren Silikonmasse in eine Kavität einer Gussform, wobei es sich bei dem ersten granularen Material um ein poröses und vorzugsweise geschäumtes thermoplastisches Kunststoffmaterial handelt; (d) Härten der Silikonmasse, um einen Prothesenkörper zu bilden; und (f) Erwärmen des Prothesenkörpers auf eine Schrumpfungstemperatur, die oberhalb des Schmelzpunktes des thermoplastischen Kunststoffmaterials liegt.

Erfindungsgemäß wird zur Porenbildung also poröses Kunststoffgranulat aus einem thermoplastischen Material verwendet, das einen geringeren Schmelzpunkt als die Silikonmatrix hat. Dadurch kann das poröse Granulat aufgeschmolzen werden, wobei die Körner dann unter entweichen des in den Poren eingeschlossenen Gases kollabieren. So entstehen Hohlräume, die das Grundmaterial signifikant weicher werden lassen, da kein Gegendruck von der Wandung des Kunststoffgranulats mehr wirkt. Bei diesem Verfahren sind eine offenporige Struktur und eine dichte Kugelpackung nicht erforderlich. Vielmehr kann die Porosität der Silikonmatrix und mithin die gewünschte Gewichtsersparnis in einem Bereich von 0% bis etwa 50% stufenlos eingestellt werden.

In einer Ausführungsform ist vorgesehen, dass es sich bei dem Kunststoffmaterial um expandiertes Polystyrol, expandiertes Polyethylen oder expandiertes Polypropylen handelt. Expandiertes Polystyrol (EPS) ist besonders bevorzugt.

Die Porosität des Kunststoffmaterials kann bei größer 0,8 oder größer 0,9 liegen. Je größer die Porosität ist, desto stärker ist die Volumenabnahme beim Aufschmelzen. Die Porosität von expandiertem Polystyrol liegt vielfach in diesen Bereichen.

Vorzugsweise ist das granulare Material zumindest weitgehend kugelförmig. Das erste granulare Material kann eine homogene Teilchengrößenverteilung aufweisen, wobei der Partikeldurchmesser von mehr als 70% und vorzugsweise von mehr als 85% der Partikel um weniger als 10% vom durchschnittlichen Partikeldurchmesser abweicht. Als Partikeldurchmesser wird in diesem Zusammenhang der volumen-äquivalente Kugeldurchmesser verstanden.

In einer Ausführungsform ist vorgesehen, dass das Kunststoffmaterial einen Schmelzpunkt von 120-180°C aufweist. Entsprechend kann die Schrumpfungstemperatur bei zwischen etwa 140-200°C liegen.

In einer Ausführungsform ist vorgesehen, dass das Verfahren ferner den folgenden, dem Schritt (b) vorgelagerten Schritt umfasst: (a) Herstellung einer Hautschicht der Brustprothese, wobei eine vernetzbare Silikonmasse in die Kavität gegossen, in Schichtform gebracht und gehärtet wird. Die Schichtdicke der Hautschicht kann beispielsweise 2-6 mm, vorzugsweise 3-5 mm betragen. Die erste Dispersion wird sodann in die bereits mit der Hautschicht belegte Kavität eingebracht. Die Hautschicht umfasst vorzugsweise gar keine dispergierten Partikel bzw. zumindest keine dispergierten Partikel aus geschäumtem thermoplastischem Kunststoffmaterial.

In einer Ausführungsform ist vorgesehen, dass das Verfahren ferner den folgenden, den Schritten (b) und (d) zwischengelagerten Schritt umfasst: (c) Herstellung einer porenfreien Abschlusszone des Prothesenkörpers.

Vorzugsweise ist dabei vorgesehen, dass eine Gegenform auf die in der Kavität befindliche erste Dispersion gelegt wird, um diese in Form zu fixieren, und die Gussform anschließend gestützt und die Silikonmasse bei gestürzter Gussform gehärtet wird. Durch das Stürzen schwimmt das erste granulare Material, das eine geringere Dichte als die Silikonmasse hat, zur nun obenliegenden Vorderseite der Gussform auf, sodass sich im nun untenliegenden rückseitigen Endbereich der ersten Dispersion eine Abschlusszone ausbildet, welche die Silikonmasse der ersten Dispersion ohne das dispergierte erste granulare Material enthält.

Alternativ kann eine porenfreie Abschlusszone beispielsweise durch Auftragen einer höherviskosen Silikonmasse auf die Rückseite des auszuhärtenden Prothesenkörpers oder durch ein Einfüllen einer niedrigviskosen Silikonmasse in einen Spalt zwischen auszuhärtendem Prothesenkörper und einer Gegenform erfolgen.

In einer Ausführungsform ist vorgesehen, dass das Verfahren ferner den folgenden, den Schritten (d) und (f) zwischengelagerten Schritt umfasst: (e) Herstellung einer Kontaktschicht der Brustprothese, wobei eine zweite Dispersion von zweitem granularem Material in einer vernetzbaren Silikonmasse derart in die bereits den Prothesenkörper enthaltende Kavität eingebracht wird, dass die Rückseite des Prothesenkörpers zumindest teilweise bedeckt wird, und die Silikonmasse der zweiten Dispersion anschließend gehärtet wird. So kann eine rückseitige Kontaktschicht hergestellt werden. Mit geeigneter Verfahrensführung lässt sich dabei eine definierte offene Porosität erreichen. Beispielsweise kann die Kontaktschicht mit dem Träger zugewandten Öffnungen gestaltet werden, indem die Gussform gestürzt wird, sodass die Kugeln auf die Oberfläche aufschwimmen, oder die Kugelpackung kann so dicht sein, dass das Granulat zur Oberfläche durchstößt. Damit wird eine belüftende Oberflächenstruktur gebildet, die ein optimiertes Mikroklima erzeugt. Die rückseitige Schicht soll dabei so dick gewählt werden, dass eine gute Belüftung entsteht, jedoch so dünn, dass sie nach dem Reinigen gut abtrocknen kann.

Durch eine porenfreie Abschlusszone des Prothesenkörpers können Verunreinigungen nicht bis in den Prothesenkörper vordringen.

Bei dem zweiten granularen Material kann es sich ebenfalls um ein geschäumtes thermoplastisches Kunststoffmaterial handeln, dessen Schmelzpunkt unterhalb der Schrumpfungstemperatur liegt. In einer Ausführungsform ist vorgesehen, dass das erste granulare Material der ersten Dispersion und das zweite granulare Material der zweiten Dispersion aus demselben Kunststoffmaterial bestehen. Dies erleichtert die Verfahrensführung, zumal in diesem Fall die optimale Schrumpfungstemperatur für beide Kunststoffmaterialien dieselbe ist. Es kann vorgesehen sein, dass das Material des ersten und zweiten Granulats überhaupt dasselbe ist, d.h., auch dieselbe Porosität etc. aufweist.

Alternativ kann das zweite granulare Material auch ein lösliches Material wie beispielsweise Zucker umfassen. Dieses Material wird vorzugsweise vor dem Erwärmen des Schrittes (f) ausgewaschen, um chemische Veränderungen, im Falle von Zucker beispielsweise ein Karamellisieren zu verhindern.

In einer Ausführungsform ist vorgesehen, dass der durchschnittliche Partikeldurchmesser des zweiten granularen Materials größer als der durchschnittliche Partikeldurchmesser des ersten granularen Materials ist. Es kann vorgesehen sein, für die Kontaktschicht größere Poren anzustreben, da dies zu einer weicheren Masse und besseren Belüftung führen kann.

Die in Schritten (a), (b) und (e) verwendete Silikonmasse kann einander chemisch entsprechen. Geeignete Silikonmassen für jeden der Schritte umfassen additionsvernetzende Zwei-Komponenten-Silikonkautschuk-Massen. Das Härten durch Vernetzen kann generell bei Raumtemperatur oder auch höheren Temperaturen erfolgen.

Der Silikonmasse zur Bildung der porenfreien Abschlusszone oder, im Falle des Vorhandenseins des Schrittes (e), der zweiten Dispersion kann gegebenenfalls ein Phasenwechselmaterial zugegeben werden, dessen Phasenübergangstemperatur nahe der Körpertemperatur liegt. Geeignete Beispiele umfassen Paraffine mit einer geeigneten Zahl an Kohlenstoffatomen, typischerweise etwa zwanzig, um einen Schmelzpunkt im gewünschten Bereich einzustellen. Durch die Zugabe des Phasenwechselmaterials speziell zu der Silikonmasse zur Bildung der porenfreien Abschlusszone oder zweiten Dispersion, aus welcher die körpernahe Schicht resultiert, wirkt das Phasenwechselmaterial dort, wo die Wirkung benötigt wird, nämlich direkt am Körper der Trägerin.

Hierin beschrieben ist weiterhin eine Brustprothese mit einem porösen Prothesenkörper, wobei die Prothese nach einem erfindungsgemäßen Verfahren hergestellt ist. Aufgrund der Herstellungsart weist der Prothesenkörper Rückstände eines geschmolzenen thermoplastischen Materials in seinen Poren auf. Diese Rückstände haben aber keinen wahrnehmbaren Einfluss auf die haptischen Eigenschaften der Prothese.

Bevorzugte Ausgestaltungen der Prothese ergeben sich aus den im Zusammenhang mit dem erfindungsgemäßen Verfahren beschriebenen Aspekten. Insbesondere kann die Prothese dann, wenn das Verfahren einen vorgelagerten Schritt (a) umfasst, eine an die Vorderseite des Prothesenkörpers anschließende, vorzugsweise Hautschicht aufweisen. Ferner kann die Prothese dann, wenn das Verfahren einen zwischengelagerten Schritt (c) umfasst, eine porenfreie rückseitige Abschlusszone des Prothesenkörpers aufweisen. Des Weiteren kann die Prothese dann, wenn das Verfahren einen zwischengelagerten Schritt (e) umfasst, eine poröse Kontaktschicht an der Rückseite des Prothesenkörpers aufweisen. Besonders bevorzugt ist es, wenn die Prothese all diese Schichten aufweist.

Weitere Einzelheiten und Vorteile der Erfindung ergeben sich aus dem nachfolgend anhand der Figuren beschriebenen Ausführungsbeispiel. In den Figuren zeigen:
- Figur 1:: eine Ausführungsform einer mittels des erfindungsgemäßen Verfahrens hergestellten Brustprothese;
- Figur 2:: eine weitere Ausführungsform einer mittels des erfindungsgemäßen Verfahrens hergestellten Brustprothese;
- Figur 3:: eine Gussform zur erfindungsgemäßen Herstellung einer Brustprothese mit in die Kavität eingelegtem Nippel-Imitat;
- Figur 4:: die Gussform der Figur 3 mit die Kavität belegender Hautschicht;
- Figur 5:: die Gussform der Figur 4 mit eingegossener erster Dispersion; und
- Figur 6:: die Gussform der Figur 5 mit eingegossener zweiter Dispersion.

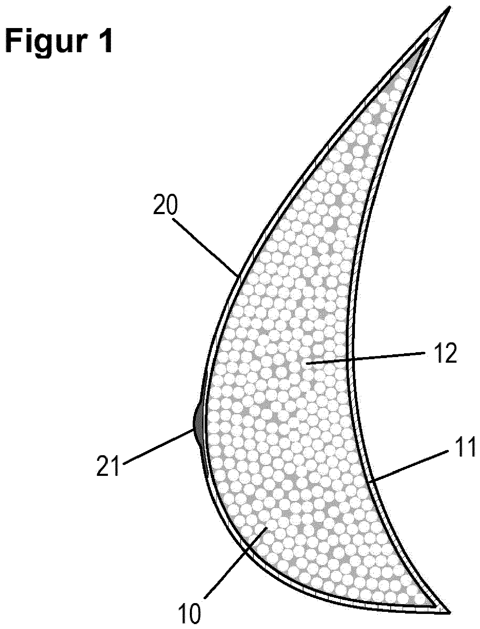

Die Figuren 1-2 zeigen zwei unterschiedliche Ausführungsformen von mittels des erfindungsgemäßen Verfahrens hergestellten Brustprothesen. Beide Prothesen umfassen einen porösen Prothesenkörper 10, der an seiner Vorderseite eine porenfreie Hautschicht 20 und an seiner Rückseite eine porenfreie Abschlusszone 11 aufweist. An der Vorderseite der Hautschicht 20 ist ferner ein Nippel-Imitat 21 angebracht. Die Schichten 10 und 20 sowie vorzugsweise auch das Nippel-Imitat 21 sind aus Silikonkautschuk gefertigt.

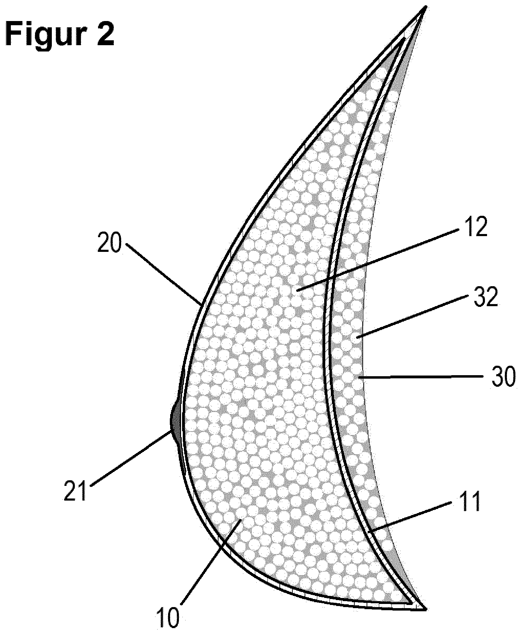

Die in Figur 2 gezeigte Brustprothese umfasst zusätzlich eine poröse Kontaktschicht 30, die an die Rückseite des Prothesenkörpers 10 anschließt. Alle Schichten 10, 20 und 30 sowie vorzugsweise auch das Nippel-Imitat 21 sind aus Silikonkautschuk gefertigt, wobei die Schichten 10 und 30 unterschiedliche Porosität und Porenstrukturen aufweisen. Die Poren 32 der porösen Kontaktschicht 30 sind dabei größer als die Poren 12 des Prothesenkörpers 10. Ferner ist die Porenstruktur der porösen Kontaktschicht 30 offenporig mit hoher Porosität nahe dem Raumfüllungsgrad einer dichtesten Kugelpackung, während der Prothesenkörper 10 eine geschlossenporige Struktur mit geringerer Porosität aufweist.

Durch die porenfreie Abschlusszone 11 des Prothesenkörpers 10 können Verunreinigungen nicht von der Kontaktschicht 30 bis in den Prothesenkörper 10 vordringen.

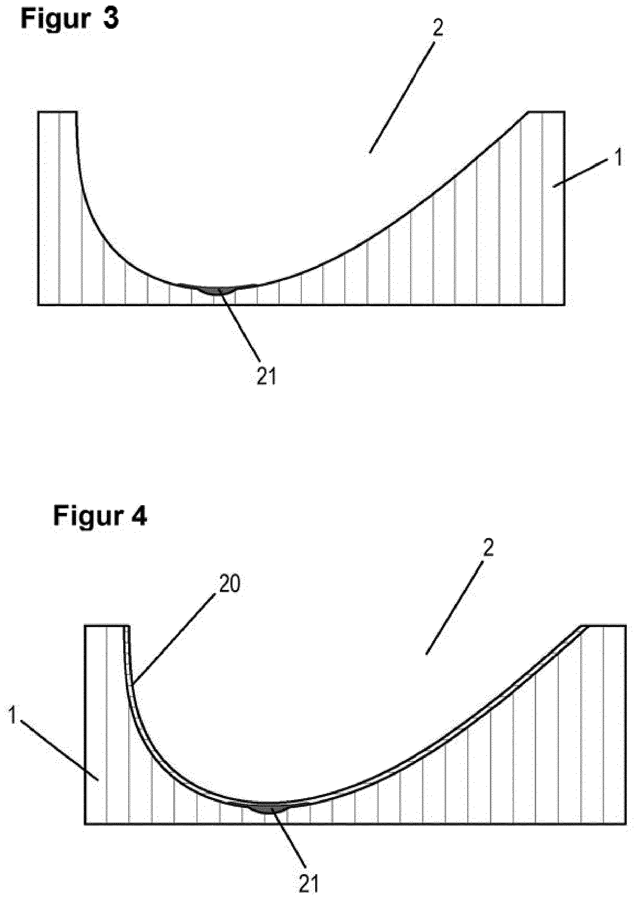

Anhand der Figuren 3-6 kann ein erfindungsgemäßes Verfahren zur Herstellung der gezeigten Prothesen erläutert werden.

Die in Figur 3 gezeigte Gussform 1 weist eine Kavität 2 auf, die korrespondierend zu einer weiblichen Brust geformt ist. Die Gussform 1 wird typischerweise aus temperaturbeständigem Material hergestellt. Die Form der Kavität 2 kann maßgefertigt oder serientauglich sein. In die Kavität 2 ist an einer entsprechenden Stelle das Nippel-Imitat 21 eingelegt. Das Imitat 21 kann aus einem speziell eingefärbten Silikonkautschuk gefertigt werden. Ebenfalls kann die Weichheit vom übrigen Silikon abweichen. Als Silikon kann z. B. Silpuran® 2420 der Fa. Wacker Chemie eingesetzt werden. Das Nippel-Imitat 11 kann durch Einbringen einer härtenden Silikonmasse in die Kavität 2 sowie deren Härten gefertigt werden. Dabei kann der entsprechende Bereich durch einen vorübergehend in die Kavität 2 eingelegten Kunststoffring begrenzt werden. Das Vernetzen kann bei Raumtemperatur oder auch höheren Temperaturen erfolgen.

Wie dies in Figur 4 zu erkennen ist, wird als nächstes die Hautschicht 20 gefertigt. Hierfür wird eine vernetzbare Silikonmasse in die offene Kavität 2 eingefüllt und diese anschließend mit einem nicht näher dargestellten Gegenstück geschlossen, um durch anschließendes Vernetzen die 2-3 mm dicke Hautschicht 20 zu erzeugen. Alternativ kann die Silikonmasse in die bereits mit dem Gegenstück geschlossene Kavität 2 eingepresst werden. Das Vernetzen kann wiederum bei Raumtemperatur oder auch höheren Temperaturen erfolgen.

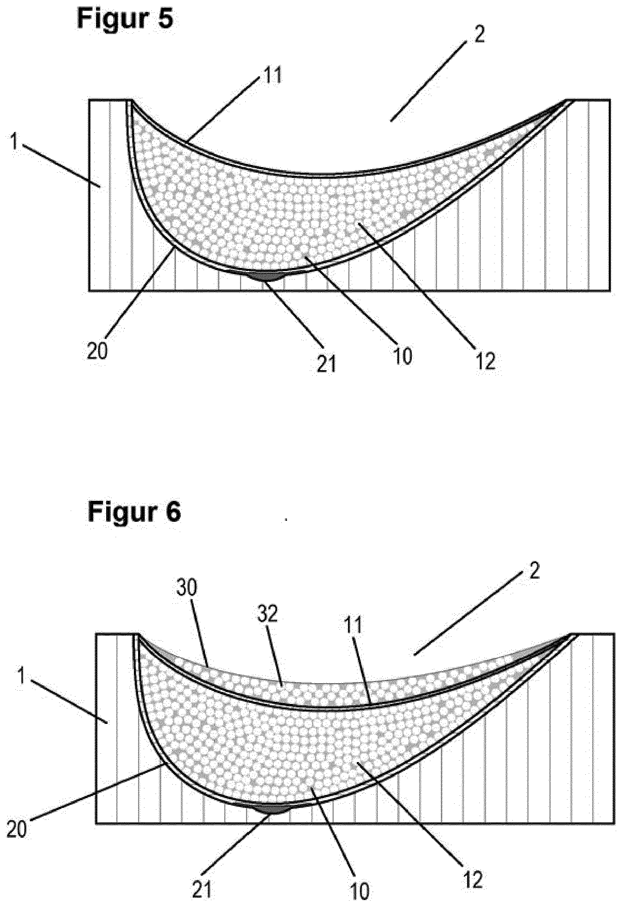

Anschließend wird, wie in Figur 5 erkennbar, eine erste Dispersion umfassend in einer vernetzbaren Silikonmasse dispergierte, expandierte Polystyrol-(EPS)-Kugeln mit einem durchschnittlichen Teilchendurchmesser von 1-2 mm in die offene Kavität 2 eingefüllt. Die Kavität 2 wird sodann mit einem nicht näher dargestellten Gegenstück geschlossen und die Silikonmasse vernetzt.

Die angereicherte Silikonmasse wird mit einer weiteren, dünnen Silikonschicht von etwa 1-2 mm Stärke überzogen, um die porenfreie Abschlusszone 11 zu bilden. Dies kann beispielsweise durch Auftragen einer höherviskosen Silikonmasse auf die Rückseite des auszuhärtenden Prothesenkörpers oder durch ein Einfüllen einer niedrigviskosen Silikonmasse in einen Spalt zwischen auszuhärtendem Prothesenkörper und Gegenstück erfolgen.

Bei der Variante gemäß Figur 2 wird noch eine weitere Schicht aufgetragen, wie dies in Figur 6 erkennbar ist. Diese Schicht resultiert in der porösen Kontaktschicht 30, die an die Rückseite des Prothesenkörpers 10 anschließt. Nämlich wird eine zweite Dispersion umfassend in einer vernetzbaren Silikonmasse dispergierte, expandierte Polystyrol-(EPS)-Kugeln mit einem durchschnittlichen Teilchendurchmesser von mehr als 2 mm in die offene Kavität 2 eingefüllt und vernetzt.

Der zweiten Dispersion wird darüber hinaus ein Paraffin mit einer durchschnittlichen Kohlenstoffzahl von etwa zwanzig zugegeben, dessen Phasenübergangstemperatur nahe der Körpertemperatur liegt. Durch die Zugabe des Phasenwechselmaterials speziell zu der zweiten Dispersion, aus welcher die poröse Kontaktschicht 30 resultiert, wirkt das Phasenwechselmaterial dort, wo die Wirkung benötigt wird, nämlich direkt am Körper der Trägerin.

Die rückseitigen Formteile können auf die Narbenkontur der Kundin abgestimmt werden, sofern es kein Serienwerkzeug ist.

Abschließend wird das rückseitige Formteil entfernt. Das Rückschrumpfen der Kunststoffkugeln erfolgt bei etwa 150-180°C für etwa 2 h. Dabei wird entweder die Form 1 zusammen mit der Prothese aufgeheizt oder die Silikonprothese wird vorher entformt. Das Aufheizen in der Form 1 hat sich bewährt, da die schwierige Entformung der noch kompakten Prothese entfällt.

Da die Silikonoberflächige etwas klebrig sein kann, was ein Einlegen in die BH-Tasche erheblich erschweren kann, wird die Silikonoberfläche noch mit einem ,Low Friction Silicone Coating MED10-6670 der Firma NuSil behandelt.

## Patentansprüche

1. Verfahren zur Herstellung einer Brustprothese, umfassend die folgenden Schritte:
(b) Einbringen einer ersten Dispersion von erstem granularem Material in einer vernetzbaren Silikonmasse in eine Kavität (2) einer Gussform (1), wobei es sich bei dem ersten granularen Material um ein poröses, und vorzugsweise geschäumtes, thermoplastisches Kunststoffmaterial handelt;
(d) Härten der Silikonmasse, um einen Prothesenkörper (10) zu bilden; und
(f) Erwärmen des Prothesenkörpers (10) auf eine Schrumpfungstemperatur, die oberhalb des Schmelzpunktes des thermoplastischen Kunststoffmaterials liegt.

2. Verfahren nach Anspruch 1, **dadurch gekennzeichnet, dass** es sich bei dem Kunststoffmaterial um expandiertes Polystyrol, expandiertes Polyethylen oder expandiertes Polypropylen handelt.

3. Verfahren nach einem der vorhergehenden Ansprüche, **dadurch gekennzeichnet, dass** das Kunststoffmaterial einen Schmelzpunkt von 120-180°C aufweist und dass die Schrumpfungstemperatur zwischen 140-200°C liegt.

4. Verfahren nach einem der vorhergehenden Ansprüche, **dadurch gekennzeichnet, dass** das Verfahren ferner den folgenden, dem Schritt (b) vorgelagerten Schritt umfasst:
(a) Herstellung einer Hautschicht (20) der Brustprothese, wobei eine vernetzbare Silikonmasse in die Kavität gegossen, in Schichtform gebracht und gehärtet wird.

5. Verfahren nach einem der vorhergehenden Ansprüche, **dadurch gekennzeichnet, dass** das Verfahren ferner den folgenden, den Schritten (b) und (d) zwischengelagerten Schritt umfasst:
(c) Herstellung einer porenfreien Abschlusszone (11) des Prothesenkörpers (10).

6. Verfahren nach einem der vorhergehenden Ansprüche, **dadurch gekennzeichnet, dass** das Verfahren ferner den folgenden, den Schritten (d) und (f) zwischengelagerten Schritt umfasst:
(e) Herstellung einer Kontaktschicht (30) der Brustprothese, wobei eine zweite Dispersion von zweitem granularem Material in einer vernetzbaren Silikonmasse derart in die bereits den Prothesenkörper (10) enthaltende Kavität (2) eingebracht wird, dass die Rückseite des Prothesenkörpers (10) zumindest teilweise bedeckt wird, und die Silikonmasse der zweiten Dispersion anschließend gehärtet wird.

7. Verfahren nach Anspruch 6, **dadurch gekennzeichnet, dass** es sich auch bei dem zweiten granularen Material um ein geschäumtes thermoplastisches Kunststoffmaterial handelt, dessen Schmelzpunkt unterhalb der Schrumpfungstemperatur liegt.

8. Verfahren nach einem der Ansprüche 6-7, **dadurch gekennzeichnet, dass** der durchschnittliche Partikeldurchmesser des zweiten granularen Materials größer als der durchschnittliche Partikeldurchmesser des ersten granularen Materials ist.

9. Verfahren nach einem der Ansprüche 5-8, **dadurch gekennzeichnet, dass** in die porenfreie Abschlusszone (11) und/oder die Kontaktschicht (30) ein Phasenwechselmaterial eingebracht wird.

## Claims

1. A method for manufacturing a breast prosthesis, comprising the following steps:
(b) introducing a first dispersion of a first granular material in a cross-linkable silicone compound into a cavity (2) of a casting mold (1), wherein the first granular material is a porous, and preferably foamed, thermoplastic material;
(d) curing of the silicone compound in order to form a prosthesis body (10); and
(f) heating of the prosthesis body (10) to a shrinking temperature which lies above the melting point of the thermoplastic material.

2. The method according to claim 1, **characterized in that** the plastic material is expanded polystyrene, expanded polyethylene or expanded polypropylene.

3. The method according to any of the preceding claims, **characterized in that** the plastic material has a melting point of 120-180°C and that the shrinking temperature lies between 140-200°C.

4. The method according to any of the preceding claims, **characterized in that** the method furthermore comprises the following step preceding step (b):
(a) manufacturing a skin layer (20) of the breast prosthesis, wherein a cross-linkable silicone compound is poured into the cavity, brought into a layer form and cured.

5. The method according to any of the preceding claims, **characterized in that** the method furthermore comprises the following step carried out between steps (b) and (d):
(c) manufacturing a pore-free terminal zone (11) of the prosthesis body (10).

6. The method according to any of the preceding claims, **characterized in that** the method furthermore comprises the following step carried out between steps (d) and (f):
(e) manufacturing a contact layer (30) of the breast prosthesis, wherein a second dispersion of second granular material in a cross-linkable silicone compound is introduced into the cavity (2) already containing the prosthesis body (10) such that the back of the prosthesis body (10) is at least partly covered, and the silicone compound of the second dispersion subsequently is cured.

7. The method according to claim 6, **characterized in that** the second granular material also is a foamed thermoplastic material whose melting point lies below the shrinking temperature.

8. The method according to any of claims 6-7, **characterized in that** the average particle diameter of the second granular material is greater than the average particle diameter of the first granular material.

9. The method according to any of claims 5-8, **characterized in that** a phase-change material is introduced into the pore-free terminal zone (11) and/or the contact layer (30).

## Revendications

1. Procédé de fabrication d'une prothèse mammaire, comprenant les étapes suivantes :
(b) introduire une première dispersion d'un premier matériau granulaire dans une composition de silicone réticulable dans une cavité (2) d'un moule (1), le premier matériau granulaire étant un matériau thermoplastique poreux, et de préférence expansé ;
(d) durcir la composition de silicone pour former un corps de prothèse (10) ; et
(f) chauffer le corps de prothèse (10) à une température de retrait qui est supérieure au point de fusion du matériau thermoplastique.

2. Procédé selon la revendication 1, **caractérisé en ce que** le matériau plastique est du polystyrène expansé, du polyéthylène expansé ou du polypropylène expansé.

3. Procédé selon l'une quelconque des revendications précédentes, **caractérisé en ce que** le matériau plastique a un point de fusion de 120-180°C et que la température de retrait est comprise entre 140-200°C.

4. Procédé selon l'une quelconque des revendications précédentes, **caractérisé en ce que** le procédé comprend en outre l'étape suivante précédant l'étape (b) :
(a) fabrication d'une couche de peau (20) de la prothèse mammaire, une composition de silicone réticulable étant versée dans la cavité, mise en forme de couche et durcie.

5. Procédé selon l'une quelconque des revendications précédentes, **caractérisé en ce que** le procédé comprend en outre l'étape suivante précédant les étapes (b) et (d) :
(c) fabrication d'une zone terminale non poreuse (11) du corps de prothèse (10).

6. Procédé selon l'une quelconque des revendications précédentes, **caractérisé en ce que** le procédé comprend en outre l'étape suivante réalisée entre les étapes (d) et (f) :
(e) fabrication d'une couche de contact (30) de la prothèse mammaire, une deuxième dispersion d'un deuxième matériau granulaire dans une composition de silicone réticulable étant introduite dans la cavité (2) contenant déjà le corps de prothèse (10) de telle sorte que la face arrière du corps de prothèse (10) soit au moins partiellement recouverte, et la composition de silicone de la deuxième dispersion étant ensuite durcie.

7. Procédé selon la revendication 6, **caractérisé en ce que** le deuxième matériau granulaire est également un matériau thermoplastique expansé ayant un point de fusion inférieur à la température de retrait.

8. Procédé selon l'une quelconque des revendications 6-7, **caractérisé en ce que** le diamètre moyen des particules du deuxième matériau granulaire est plus grand que le diamètre moyen des particules du premier matériau granulaire.

9. Procédé selon l'une quelconque des revendications 5 à 8, **caractérisé en ce qu'**un matériau à changement de phase est introduit dans la zone terminale non poreuse (11) et/ou la couche de contact (30).
